# EUROPEAN PATENT SPECIFICATION

(11) **EP 2 963 107 B1**
(45) Date of publication and mention of the grant of the patent: **15.11.2017**
(21) Application number: 15174149.3
(22) Date of filing: 26.06.2015
(51) Int. Cl.: C12M 1/00, C10L 3/08, C10L 3/10, B01D 53/04, B01D 53/047

(54) **PROCESS FOR REFINING A BIOMETHANE BIOGAS STREAM AND RELATIVE APPARATUS FOR THE IMPLEMENTATION THEREOF**
VERFAHREN ZUR VEREDELUNG EINES BIOMETHANBIOGASSTROMS UND ZUGEHÖRIGE VORRICHTUNG ZUR IMPLEMENTIERUNG DAVON
PROCEDE DE RAFFINAGE D'UN FLUX DE BIOGAZ ET APPAREIL ASSOCIE POUR LA MISE EN OEUVRE DE CELUI-CI

(30) Priority: 30.06.2014 IT MI20141190
(43) Date of publication of application: 06.01.2016
(73) Proprietor: Ricerca Sul Sistema Energetico - RSE S.p.A., 20134 Milano (IT)
(72) Inventor: NOTARO, Maurizio, 24043 Caravaggio (BG) (IT); MAZZOCCHI, Luigi, 20060 Cassina de'Pecchi (MI) (IT); CASTELLAZZI, Paola, 20861 Brugherio (MB) (IT); CAVALLARI, Andrea, 20134 Milano (MI) (IT)
(74) Representative: Bottero, Carlo

(56) References cited:
- EP-A1- 1 634 946
- EP-A1- 2 638 951
- US-A1- 2002 069 838
- US-A1- 2011 023 497
- US-A1- 2012 024 157
- US-A1- 2013 209 338

## Description

The present invention relates to a process for upgrading a stream of biogas to biomethane and the relative apparatus for its implementation.

As is known, biogas is a mixture of gases produced by the bacterial digestion, under anaerobic conditions, of organic residues, such as vegetable and animal residues. Biogas is mainly composed of methane, CO₂ and, to a minimum extent, of other compounds, such as H₂S, NH₃, water vapour and particulate.

High quantities of biogas are produced, for example, in landfills for solid urban waste, in sewage treatment plants (by the digestion of sewage sludge) or in specific biogas production plants by means of anaerobic digesters of organic residues.

Biogas is typically exploited in production sites as fuel for feeding internal combustion engines capable of producing heat and electric energy (cogeneration engines).

A more efficient use of biogas, however, is represented by its upgrading to methane. The biogas is purified, through the upgrading process, of most of the contaminating compounds that can be harmful for the environment or the treatment plant itself (e.g. H₂S that can cause problems of erosion) and its calorific value is increased due to the removal of CO₂ (the content of CH₄ in biomethane is generally higher than approximately 97% v/v). The upgrading process therefore allows a more valuable fuel to be obtained, with characteristics suitable for use as automotive fuel and, more generally, as biofuel.

The commercial exploitation of biomethane, however, is strictly connected to the possibility of cost-effectively obtaining biomethane with characteristics that are such as to be able to be introduced into natural gas transport and distribution networks. Through these networks, in fact, biomethane can be transported also at considerable distances with respect to the production site, to be subsequently used in large high-efficiency combustion plants (e.g. cogeneration or regeneration plants), with significant advantages from an environmental point of view.

Various upgrading processes of biogas to biomethane are known and used in the state of the art. These processes, however, are generally characterized by a certain plant complexity, significant energy consumptions and high investment and management costs of the plants.

The main upgrading technologies of biogas applied on an industrial scale are: adsorption on solid sorbents (e.g. activated carbons), physical absorption in water (so-called water scrubbing) or organic solvents and chemical absorption in reactive solutions.

The most widely-used upgrading process on an industrial scale is currently water scrubbing. In this process, a stream of biogas is fed to the bottom of a water column. As the solubility of CO₂ in water is greater than the solubility of methane, the CO₂ is separated from the biogas, whereas the purified stream of biomethane leaves the head of the column. In addition to producing high-purity biomethane, this process has the advantage of also removing the H₂S and particulate possibly present in the biogas. Water scrubbing, however, requires high operating pressures and is strongly jeopardized by the high energy consumptions for the regeneration of the sorbent means. Furthermore, due to the absorption of H₂S, water scrubbing creates significant problems of corrosion damaging the upgrading plant.

Upgrading techniques based on physical absorption of CO₂ in organic solvents (e.g. polyethylene glycol) or of the chemical type in reactive solutions (e.g. aqueous solutions of amine compounds) exploit the greater solubility of CO₂ in these solvents and solutions with respect to water. The use of these techniques offers the possibility of producing upgrading plants having reduced dimensions. In the case of physical absorption in organic solvents, however, although more compact, the plants must be in any case operated at high pressure to guarantee satisfactory separation efficiencies of the CO₂. In the case of chemical absorption in amine solutions, the high reactivity and selectivity of the amine with respect to CO₂, allows the process to also be carried out at low pressure, against however the high thermal requirements necessary for regenerating the solution. Further drawbacks of these plants are the high energy consumption for regenerating the liquid sorbent, the necessity of reintegrating or substituting said sorbent and the necessity of previously removing the water present in the biogas to reduce the contamination of the sorbent and to avoid diluting the solution.

Adsorption processes of CO₂ on solid sorbents typically use activated carbons or molecular sieves (e.g. zeolites) as sorbent materials. Solid sorbents are capable of selectively adsorbing CO₂ and possibly other small-sized gaseous molecules (e.g. O₂ and N₂), allowing a high-purity biomethane to be produced. The adsorption process is favourable at high pressures (e.g. 7 bar) whereas the desorption process, necessary for regenerating the sorbent, is carried out at atmospheric pressure or under a slight vacuum (adsorption effected in so-called Pressure Swing Adsorption (PSA) mode. Although the PSA process has reduced energy consumptions, it has, in any case, an overall production yield lower than that of processes using liquid sorbent means and entails high investment and management costs of the plants.

Document EP 1634946 A1 relates to a process to upgrade a biogas stream characterized in that the methane-containing emissions which are generated during the upgrading process are used to produce exclusively thermal energy which is used in the biogas production process. The recovery of the emissions produced during the biogas upgrading would avoid dispersion of methane (a greenhouse gas) in the atmosphere.

EP2638951 discloses a process for removing CO₂ from a mixture which contains it, for example a biogas, comprising: a first phase of removal of CO₂ from said mixture by physical adsorption in a liquid medium so as to produce a pre-purified gaseous mixture; a second phase of removal of CO₂ from said pre-purified mixture through adsorption on a solid substrate, for example through a PSA process, to separate the remaining part of CO₂ from the pre-purified mixture.

In the light of the above state of the art, the Applicant has set the objective of finding a new upgrading process of biogas to biomethane which does not have the disadvantages of the processes of the known art.

An objective of the present invention, in particular, is to provide an upgrading process of biogas to biomethane and a relative apparatus for its implementation, which is simple to effect, has a high removal efficiency of the CO₂, low energy consumptions and implies reduced investment and management costs of the plants.

In the light of these objectives and yet others that will appear more evident hereunder, in accordance with a first aspect of the present invention, a process is provided for upgrading a stream of biogas, comprising at least methane and CO₂, to biomethane, said process comprising at least the following steps:
a) supplying a part of said biogas stream to a cogeneration system and producing a heating fluid and electric energy;
b) supplying said electric energy at least to cooling means and producing a cooling fluid;
c) supplying the remaining part of said biogas stream to a treatment system comprising at least a first and a second treatment unit each comprising at least one solid sorbent;
   in said treatment system occurring, for at least a certain operating period under regime conditions, that:
   - the CO₂ present in said remaining part of said biogas stream is adsorbed on the solid sorbent of said first treatment unit with the formation of a stream of biomethane;
   - the solid sorbent of said second treatment unit is subjected to regeneration with the formation of a stream of desorbed CO₂ and a regenerated solid sorbent;
   said heating fluid and said cooling fluid being used for regulating the temperature of said solid sorbents.

According to another aspect, the present invention relates to an apparatus for upgrading a stream of biogas, comprising at least methane and CO₂, to biomethane, said apparatus comprising:
- at least one cogeneration system for producing a heating fluid and electric energy, said cogeneration system being fed with at least one part of said stream of biogas;
- cooling means for producing a cooling fluid electrically fed by said cogeneration system;
- at least one treatment system comprising at least a first and a second treatment unit connected in parallel to each other, each comprising at least one solid sorbent; said first and second treatment unit being connected to said cogeneration system for receiving said heating fluid and to said cooling means for receiving said cooling fluid;

- means for feeding biogas for supplying the remaining part of said biogas stream to at least said first and second treatment units;
- means for feeding a thermal fluid for supplying said heating fluid and/or said cooling fluid to at least said treatment units so as to regulate the temperature of the respective solid sorbents.

The upgrading process of biogas to biomethane according to the present invention is based on the use of solid sorbents for capturing the CO₂ present in the biogas stream, thus obtaining a stream of biomethane. According to the present invention, a small fraction of the starting biogas stream is used for producing energy, in the form of heat and electricity; said energy is advantageously used for satisfying the energy consumptions of at least some, more preferably all, of the equipment used for implementing the process itself. The choice of effecting the upgrading treatment by adsorption on solid sorbents - which can be regenerated with a lower energy consumption - and producing the energy necessary for effecting said treatment with part of the starting biogas stream, allows biomethane to be obtained, having suitable characteristics for entering the general methane distribution network in a simple, effective and economically advantageous way, with respect to both the management of the process and the plant investment costs.

The process and apparatus according to the present invention therefore significantly increase the exploitation potentiality of biomethane in high-efficiency combustion plants, such as, for example, cogeneration plants (production of thermal energy and electricity) and trigeneration plants (production of thermal energy, cooling energy and electricity), thus making the production of methane from renewable sources more advantageous.

The process according to the present invention is effected using an apparatus comprising at least two treatment units, each of which comprises at least one solid sorbent capable of capturing the CO₂ present in the biogas, producing biomethane. The process is carried out, periodically varying the temperature of the sorbent and the pressure inside each treatment unit so as to obtain a "Temperature and Pressure Swing Adsorption, *TPSA"* of the CO₂. The adsorption of CO₂ on the sorbent is in fact carried out at low temperature values and at atmospheric pressure of the biogas, whereas the desorption of CO₂ from the sorbent, with a consequent regeneration of the sorbent itself, is effected at high temperature values and at low pressure (e.g. under vacuum). Optionally, a flow of vapour or inert gas can also be used as stripping stream for favouring the regeneration process of the sorbent.

The adsorption phases of CO₂ and regeneration of the sorbent are carried out in sequence on each treatment unit and repeated cyclically. At a given operating moment, under regime conditions, of the upgrading apparatus, the adsorption-regeneration cycles are carried out so that, when the adsorption is effected on a first treatment unit, the regeneration is effected on a second or further treatment unit, thus guaranteeing the implementation of the upgrading process in continuous.

The characteristics and advantages of the present invention will appear more evident from the following illustrative and non-limiting description, referring to the enclosed schematic drawings, in which:
- figure 1 is a scheme of a possible embodiment of the process and apparatus according to the present invention;
- figure 2 is a schematic representation of a transversal section of a preferred embodiment of a treatment unit.

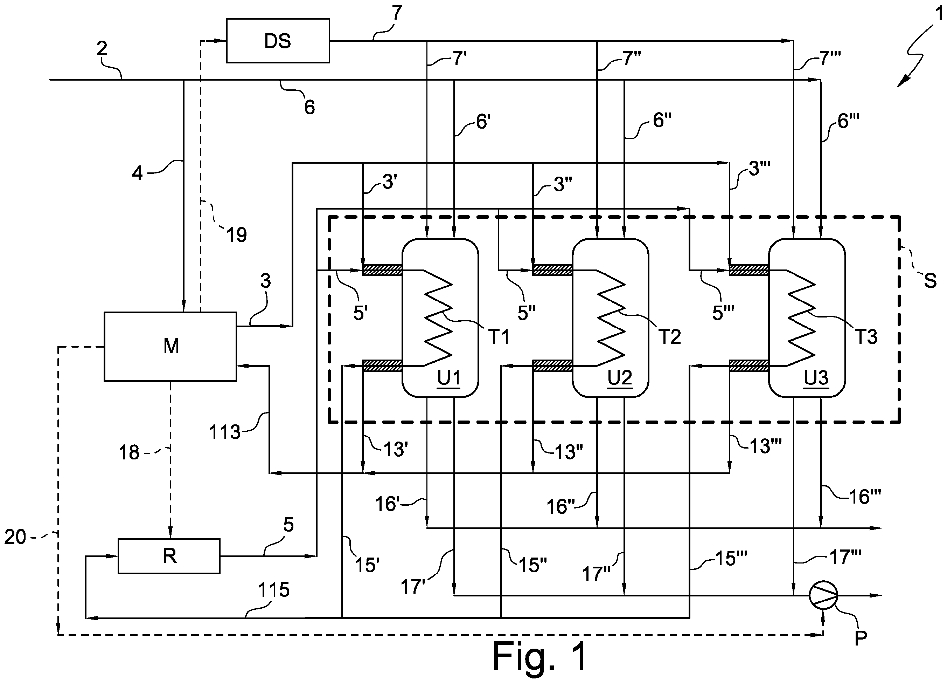

With reference to figure 1, the number 1 indicates as a whole an apparatus for upgrading a stream of biogas according to the present invention.

The apparatus 1 comprises at least one cogeneration system M for producing heat and electric energy. The heat produced is used for heating a thermal fluid (for example, water or oil), so as to form a heating fluid 3 leaving the above system M. The system M can be fed, through supply means (not shown in figure 1), with a part 4 of the starting biogas stream 2. The system M is a conventional cogeneration system, comprising, for example, at least one internal combustion engine which can use biogas as fuel, at least one electric generator which, when moved by said engine, is capable of producing electricity, and one or more heat recovery units (e.g. heat exchangers) through which a heating fluid is produced.

The apparatus 1 also comprises cooling means R for cooling a thermal fluid (for example, water or oil) so as to produce a cooling fluid 5 leaving said cooling means R. The cooling means R are connected to said cogeneration system M by which they are electrically fed by the electric energy (connection 18) produced by the same. For the purposes of the present invention, an air heater can be used as cooling means, wherein the cooling fluid is cooled by indirect heat exchange with air.

The apparatus 1 also comprises a biogas treatment system S comprising at least a first and a second treatment unit, indicated in figure 1 as U1 and U2, respectively, connected to each other in parallel.

In the preferred embodiment of the invention shown in figure 1, the apparatus 1 also comprises a third treatment unit U3, preferably having the same structural characteristics as the above units U1 and U2, and connected in parallel with respect to the same.

Each of said units U1-U3 comprises: (i) at least one solid sorbent and (ii) temperature regulation means T1, T2 and T3 of said sorbent material. The above treatment units U1-U3 are connected to said cogeneration system M so as to be able to receive said heating fluid 3 by means of streams 3'-3''', and to said cooling means R for receiving said cooling fluid 5 through streams 5'-5'''. The heating fluid 3'-3''' and the cooling fluid 5'-5''' can exchange heat indirectly with the sorbent present inside the treatment units U1-U3, so as to heat or cool said sorbent in accordance with the various steps of the process of the present invention.

The remaining part 6 of the stream of biogas 2, i.e. the fraction of biogas not fed as fuel to the cogeneration system M, is subjected to the upgrading process by feeding it to the treatment system S. For this purpose, the treatment system S is connected to feeding means of the biogas (not shown in the figure) capable of supplying the remaining part 6 of said starting stream of biogas 2 to each treatment unit, as streams 6', 6'' and 6'''.

In the treatment units U1-U3, the streams of biogas 6'-6''' come into contact with the solid sorbent so that the CO₂ present in the biogas remains adsorbed on the surface of the sorbent, whereas the remaining gaseous stream, prevalently composed of methane (stream of biomethane), exits the treatment units as stream 16' -16'''.

The adsorption of the CO₂ is preferably effected on a solid sorbent in granular form. The granules of sorbent are more preferably arranged in the treatment unit in the form of a fixed bed.

During the various process phases, the temperature of the sorbent can be varied by means of the above temperature regulation means T1-T3. The temperature of the sorbent is preferably regulated by means of indirect heat exchange between the sorbent and heating liquid 3'-3''' or between the sorbent and cooling liquid 5'-5'''. Once the heating liquid 3'-3''' has transferred heat to the sorbent (for example during the desorption phase of the CO₂), it exits the treatment units as stream 13'-13'''. Said cooling fluid 13'-13''', which has a lower temperature with respect to that of the heating fluid 3'-3''' at the inlet, is recirculated to the cogeneration system M (stream 113), where it is re-heated and then re-introduced into the apparatus as heating fluid 3.

Analogously, when the sorbent must be cooled (e.g. CO₂ adsorption phase and cooling phase of the regenerated sorbent), this is put into contact (indirect) with the cooling fluid 5'-5''' so as to remove the heat. The cooling fluid 5'-5''' leaves the treatment units as stream 15'-15''' at a higher temperature than that of the cooling fluid 5'-5''' at the inlet. It is therefore fed to the cooling means R (stream 115) to be cooled again by these and then recirculated in the apparatus as cooling fluid 5.

The apparatus 1 can also be advantageously equipped with a device DS for generating a gaseous stripping stream 7. The above device DS is connected to the treatment units U1-U3 so as to be able to feed said stripping stream (streams 7', 7" and 7''') on the sorbent. Said device DS is advantageously connected to said cogeneration system M by which it is fed electrically (connection 19).

The stripping stream 7'-7''' is used for assisting the regeneration of the sorbent, for example, when this has reached the maximum adsorption capacity of CO₂ and is therefore subjected to regeneration. The use of a stripping stream combined with a temperature increase of the sorbent favours the desorption of the CO₂ and its removal from the sorbent in the form of a gaseous stream 17'-17'''. As the sorbent thus regenerated is substantially free of CO₂, it can be used for a new adsorption cycle.

The stripping stream 7'-7''' can, for example, be a stream of vapour or inert gas (e.g. nitrogen). The device for generating the stripping stream is, for example, a nitrogen generator starting from compressed air. Examples of these generators, known in the state of the art, are nitrogen generators that operate by pressure swing adsorption (PSA).

The gaseous stream of CO₂ formed in the treatment unit U1-U3 during the desorption, is mixed with the gaseous stripping stream 7'-7''', when present, leaving the treatment unit U1-U3 as stream 17'-17'''.

The apparatus 1 can also envisage the presence of a vacuum pump P connected to said treatment units U1-U3 to put the interior of the same unit in depression, for example during the desorption step of the CO₂. The reduced pressure, in fact, favours the desorption of CO₂ and therefore the regeneration of the solid sorbent. Said vacuum pump P is advantageously connected to said cogeneration system M by which it is fed electrically (connection 20).

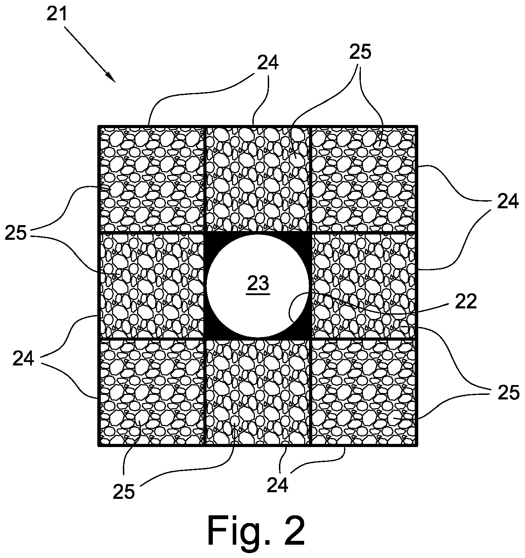

In a preferred embodiment of the present invention, the treatment units can comprise a tube-bundle structure as schematically illustrated in figure 2.

According to figure 2, a treatment unit 21 comprises at least:
- a main tube 22 through whose cavity 23 a thermal fluid (e.g. a heating fluid or cooling fluid) flows,
- a plurality of auxiliary tubes 24, optionally parallel to each other and to the main tube 22, in contact with said main tube 22, each auxiliary tube 24 containing at least one solid sorbent 25.

The above structure of the treatment unit maximizes the heat-exchange surface with respect to the sorbent mass that can be placed in its cavities.

The above main tube 22 preferably has a circular transversal section. The above auxiliary tubes 24 preferably have a rectangular, more preferably square, transversal section.

The above treatment unit 21 substantially acts as an indirect tube-bundle heat exchanger, wherein the heating or cooling fluid that flows in the main tube 22 exchanges heat through the walls of said main tube 22 with the solid sorbent 25 present in the auxiliary tubes 24. The stream of biogas to be treated, on the other hand, flows in the auxiliary tubes 24 in direct contact with the solid sorbent 25, as also the desorbed stream of CO₂ and the possible stripping stream used in the desorption phase.

For the purposes of the present invention, various types of solid sorbents, known in the state of the art, can be used.

The solid sorbent can be selected, for example, from the following types of sorbent: i) carbon molecular sieves, (ii) activated carbon, (iii) one or more amine compounds, preferably alkanolamines, supported on a solid substrate, iv) one or more carbonate salts.

In a particularly preferred embodiment, the solid sorbent comprises at least one alkanolamine having formula (I) OH-R₁-N-R₂R₃, wherein:
- R₁ is a group -(CH₂)ₙ wherein n is an integer in the range from 2 to 4,
- R₂ and R₃, the same or different from each other, are selected from: a hydrogen atom, a C₁-C₄ alkyl group or a -(CH₂)ₙOH group wherein n is an integer from 2 to 4;
said alkanolamine being supported on a solid substrate, such as alumina, silica, alumino-silicates or a combination thereof.

Preferred examples of alkanolamines that can be used for the purposes of the present invention are: monoethanolamine (MEA), diethanolamine (DEA), methyldiethanolamine (MDEA), diisopropanolamine (DIPA) and diglycolamine (DGA), more preferably diethanolamine.

The alkanolamines are preferably selected from mono- and di- alkanolamines.

Further amine compounds that can be used for the purposes of the present invention are amino acids.

Examples of carbonate salts that can be used as sorbents are salts of alkaline and alkaline-earth metals.

The solid substrate preferably has a high specific surface area (e.g. BET surface > 100 m²/g, preferably > 200 m²/g) and a high porosity (e.g. BJH porosity > 0.5 ml/g).

The carrier is preferably alumina, for example in granular or pelletized form.

The quantity of alkanolamine present on the solid substrate can vary, for example, from 5% to 60% by weight with respect to the total weight of the sorbent composition (substrate + alkanolamine).

Alkanolamines supported on alumina or another inert substrate can be prepared according techniques known in the art. The alkanolamines can be deposited, for example, on a solid substrate, by dispersing said substrate in an aqueous solution (or in an organic solvent or mixture of the two) of alkanolamines and subsequently heating the substrate impregnated with solution to evaporate the solvent.

According to the present invention and with reference to figure 1, the upgrading process of biogas, in a regime operating period, can be carried out as follows.

The starting stream of biogas 2 is divided into a first fraction 4 to be used as fuel for the cogeneration system M and a remaining fraction 6 to be subjected to upgrading to obtain biomethane. The volume ratio between the fraction of biogas 2 used as fuel and the fraction 6 subjected to treatment can vary, in particular, in relation to the adsorption capacity and thermal capacity of the sorbent. The above ratio generally ranges from 1:3 to 1:20. In the case of sorbents based on alkanolamines supported on solid substrates, for example, the above ratio varies within the range of 1:4 to 1:6, and is preferably about 1:5.

Before being sent to the upgrading process, the fraction of biogas 6 can be advantageously subjected to one or more pre-treatment steps to remove H₂S, siloxane compounds and possible humidity.

The fraction of biogas 6 to be treated is fed to a first treatment unit U1 as stream 6'. The CO₂ present in said stream 6' is adsorbed on the solid sorbent contained in said first treatment unit U1 with the formation of a stream of biomethane 16'. The adsorption is effected at a first temperature T_{ads}, lower with respect to a second temperature T_{des} at which the subsequent regeneration of the sorbent is carried out. The adsorption of the CO₂ is preferably carried out at a temperature T_{ads} of the sorbent within the range of 20-60°C. During the adsorption, in addition to CO₂, the sorbent may also capture small quantities of other gases (for example, N₂ and O₂). The quantity of further gases adsorbed mainly depends on the type of sorbent used, and also on the temperature and pressure conditions adopted.

The adsorption phase is preferably carried out by feeding the stream of biogas onto the sorbent at atmospheric pressure.

As the adsorption process is exothermic, the cooling fluid 5 is preferably fed, during the adsorption phase, to the treatment unit U1 in order to remove the heat produced by the adsorption reaction between the CO₂ and sorbent and maintaining the latter at the adsorption temperature T_{ads}.

The above stream 5' exits the unit U1 as stream 15' at a higher temperature with respect to the inlet temperature. Said outgoing stream 15' is recirculated to the cooling means, where it is cooled and then re-used as cooling fluid 5.

The stream of biogas 6' is preferably left to flow continuously inside the treatment unit U1 in contact with the sorbent for as long as the latter is still able to effectively capture the CO₂ present in the biogas and the biomethane that exits the column has an adequate degree of purity. The adsorption phase can be stopped, for example, when the stream of biomethane 16' that exits the unit U1 contains a quantity of CO₂ lower than or equal to 2% v/v and therefore approximately equal to that present in natural gas.

When the adsorption phase is considered complete, the feeding of the stream of biogas 6 to the above first unit U1 is interrupted and diverted to a second treatment unit U2 (stream 6") to continue the upgrading treatment. In the second unit U2, which comprises at least one fresh or regenerated solid sorbent, the CO₂ is separated from the stream of biogas 6" by adsorption on the sorbent with the formation of a stream of biomethane 16" leaving the unit U2. The adsorption phase is preferably carried out in the unit U2, under the same temperature and pressure conditions indicated for the adsorption process in the unit U1.

While the CO₂ is being separated from the stream of biogas 6" in the unit U2, the unit U1 is subjected to a regeneration treatment of the sorbent. Said treatment envisages the desorption of the CO₂ previously adsorbed and the formation of a regenerated solid sorbent, which can be used for a new adsorption cycle.

The regeneration phase preferably comprises heating the sorbent to a temperature T_{des}, higher than the temperature T_{ads} at which the adsorption was effected, and the consequent formation of a gaseous stream 17' substantially comprising CO₂ and other possible gases adsorbed which can be desorbed at the temperature T_{des}. For this purpose, the feeding of the cooling fluid 5' to the first unit U1 is interrupted and a stream of heating fluid 3' coming from the cogeneration system M is fed, instead of said cooling fluid 5'. The heating stream 3' transfers heat to the sorbent, bringing it to the temperature T_{des} and maintaining it at this temperature during the desorption phase. Said heating stream 3', which leaves the unit U2 as stream 13' having a lower temperature with respect to the temperature at the inlet, is recirculated to the cogeneration system M, where it is heated and then re-used in the treatment system S as heating fluid 3.

The desorption of the CO₂ is preferably effected at a temperature of the sorbent higher than 60°C and lower than or equal to 120°C.

In order to favour the desorption of CO₂ by the solid sorbent and its removal from the unit U1, the pressure inside said unit U1 is preferably reduced to below atmospheric pressure. The desorption is preferably carried out at a pressure lower than 800 mbar, even more preferably ranging from 50 mbar to 800 mbar.

These reduced pressure conditions can be created, for example, through a vacuum pump P, connected to the treatment units U1-U3.

In order to accelerate the regeneration of the sorbent and favour the desorption of the CO₂, a gaseous stripping stream 7' can also be fed to the unit U1. Said stripping stream 7', which can, for example, be an inert gas or vapour, comes into contact with the sorbent, favouring the removal and expulsion of the CO₂ from the unit U1 as stream 17'.

It has been observed that a significant increase in the regeneration degree of the sorbent can be obtained, when the desorption is carried out at reduced pressure with respect to atmospheric pressure, using a stripping stream having a flow-rate ranging from 5% to 25% by volume with respect to the average flow-rate in Nl/h of CO₂ desorbed by the sorbent in a desorption cycle (average flow-rate = [adsorption capacity of the sorbent (g CO₂/Kg sorbent)/desorption duration (h)* M.W.] * amount of sorbent used in the unit (kg sorbent) *R*273, 16°K/1 atm wherein M.W. is the molecular weight of the CO₂ equal to 44 (g/moles) and R the constant of the gases equal to 0.0821(l*atm/°K*moles).

At the end of the desorption, the regeneration phase comprises a cooling step of the sorbent from the desorption temperature T_{des} up to a temperature T_{ads} adequate for re-effecting the adsorption of CO₂. The sorbent is preferably cooled to a temperature within the range of 20°C-60°C. For this purpose, the feeding of the heating fluid 3' to the first unit U1 is interrupted, through feeding means of a thermal fluid (not shown in figure 1), and the stream of cooling fluid 5' coming from the cooling means R, is fed instead of the heating fluid 3'. The cooling stream 5' cools the sorbent up to the adsorption temperature T_{ads}, and then exits the unit U1 as stream 15' at a higher temperature with respect to the temperature at the inlet. The stream of cooling fluid 15' that exits the unit U1 is recirculated to the cooling means R, where it is cooled and then re-used in the treatment system S as cooling fluid 5. Once the sorbent has been cooled to the adsorption temperature T_{ads}, the unit U1 becomes available again for treating the stream of biogas 3 to be upgraded to biomethane.

The process according to the present invention allows high-purity biomethane to be obtained (methane content higher than 97% v/v), which can therefore be introduced into a natural gas distribution network, after compression (typically, at 7 bar).

The process is also characterized by lower energy consumptions (associated in particular with the choice of a solid sorbent means), which are completely covered by the production of thermal energy and electricity of a cogeneration system fed with a fraction of biogas to be treated.

Furthermore, the process is characterized by management simplicity, plant investment costs comparable to those of the most widely-used upgrading technologies in the art, and much lower operating costs, thanks to the lower energy consumptions.

The process according to the invention advantageously enables the upgrading of a stream of biogas to biomethane in continuous, using a treatment system with at least two treatment units connected to each other in parallel. In fact, by operating the system described above so that the adsorption phase of CO₂ is carried out in a first treatment unit, while the sorbent is being regenerated in the second treatment unit, the flow of the biogas stream can be cyclically diverted from one unit to the other, thus guaranteeing an upgrading treatment of biogas which is continuous with time.

It has been observed that, in order to allow the above continuity in the upgrading process, the adsorption phase must have a longer or equal duration than the sum of the durations of the regeneration and cooling phases.

In a particularly preferred embodiment of the present invention, the treatment system comprises at least three treatment units connected in parallel to each other and the process is effected so that:
- the duration of the adsorption, desorption and cooling phases is substantially the same (e.g. 1 hour);
- each of these phases, at a given operating moment, under regime conditions, is effected on a different treatment unit.

For this purpose, the time necessary for effecting each of the above phases can be regulated, by suitably selecting the solid sorbent, the temperature and pressure values for the adsorption and desorption phases, and possibly using a stripping stream during the regeneration of the sorbent.

The following embodiment example is provided for purely illustrative purposes of the present invention and should in no way be considered as limiting its protection scope defined by the enclosed claims.

### EXAMPLE

The effectiveness of the process, object of the present invention, was evaluated on a laboratory plant using a sorbent consisting of diethanolamine (DEA) supported on pelletized alumina spheres (average diameter of the pellets equal to 3 mm, BET specific surface area equal to 270 m²/g, porosity BJH equal to 1 cc/g). The DEA content of the sorbent was equal to 36% by weight with respect to the weight of the sorbent. The sorbent was prepared by suspending 96 g of pelletized alumina in a solution containing 54 g of DEA dissolved in 200 ml of methanol and 10 ml of water. The solution was kept under stirring for 1 h at 50°C and subsequently dried under vacuum inside a rotavapor immersed in a water bath maintained at a temperature of 50°C. The temperature of the bath was then increased to a value of 90°C, under a constant vacuum, so as to guarantee the complete elimination of the solvent from the pores of the carrier.

The capturing capacity of CO₂ on the part of the sorbent was evaluated on a laboratory plant operating with a gaseous stream containing 60% v/v of CH₄ and 40% v/v of CO₂ under the following conditions:
- flow-rate of the stream of CH₄/CO₂ equal to 250 Nl/h;
- quantity of sorbent equal to 650 g;
- adsorption temperature T_{ads} equal to 40°C;
- adsorption pressure equal to 1 atm;
- desorption temperature T_{des} equal to 70°C;
- desorption pressure equal to 100 mbar;
- stripping stream of N₂ 1.2 Nl/h.

Under the above conditions, the capturing capacity of the sorbent proved to be 33 g CO₂/kg sorbent.

On the basis of this capturing capacity, the energy and production efficiency of an upgrading apparatus comprising three treatment units, as represented in figure 1, operating in continuous, can be estimated. For this purpose, the following conditions were considered:
1) flow-rate of the stream of gas to be treated 500 Nm³/h (CH₄:CO₂ equal to 60:40 (v/v));
2) absorption at atmospheric pressure and 40°C;
3) regeneration at 70°C, 100 mbar and with 40 Nm³/h of stripping stream of N₂;
4) adsorption capacity of CO₂ equal to 33 g CO₂/kg sorbent;
5) bulk density of the sorbent equal to 667.4 kg/m³;
6) duration of the single adsorption, regeneration and cooling phases equal to 1 hour.

On the basis of the absorption capacity of the CO₂ and bulk density of the sorbent, it can be assumed that each unit must contain 12 tons of sorbent to be able to treat 500 Nm³/h of biogas in continuous. Said quantity of sorbent can be heated in 1 hour up to a temperature of 70°C, feeding a flow-rate of hot water (90°C) equal to 6.1 kg/s, to the treatment unit. Said flow-rate of hot water can be generated by a cogeneration engine having the project characteristics indicated in Table 1 below.

**Table 1 - Project data of the cogeneration engine**

| **ENGINE DATA** | |
|---|---|
| Input power [kW] | 629 |
| Biogas flow-rate [Nm³/h] | 93.4 |
| Lambda | 1 |
| H₂O flow-rate [kg/s] | 6.1 |
| Temperature of hot water generated [°C] | 90 |
| Maximum ΔT on water [°C] | 20 |
| Thermal power [kW] | 404 |
| Electric power [kW] | 226 |

The heat exchanger integrated in the cogeneration engine produces a water flow-rate equal to 6.1 kg/s at a temperature of 90°C. By sending the water at 90°C inside a treatment unit according to figure 2, the sorbent can be heated to a temperature of 70°C in about 0.5 h.

Once the sorbent has reached the desired temperature of 70°C, the desorption of CO₂ can be completed within 1 h, by depressurizing the treatment unit at 100 mbar by means of a vacuum pump and flushing the same unit with the nitrogen stripping stream. The stripping stream can be produced by a commercial nitrogen generator of the PSA type, which uses molecular sieves capable of withholding and selectively separating the oxygen present in the air. The lack of oxygen in the stripping stream ensures the absence of degradation phenomena of the sorbent attributable to amine oxidation reactions. The nitrogen generator is capable of treating a flow-rate of 50.6 Nm³/h of compressed air at about 10 bar.

Cold water at a temperature of 20°C can be used for cooling the treatment unit from the regeneration temperature to the adsorption temperature and disposing of the heat produced by the exothermic reaction, from the unit. The water flow-rate necessary for carrying all the sorbent from 70°C to 40°C in 1 h is equal to about 6.5 kg/s. It has been calculated that the cooling water leaves the treatment unit at a maximum temperature of 35°C. An air heater capable of removing about 375 thermal kW, can be used for bringing it back to a temperature of 20°C.

Against a feeding flow-rate of 500 Nm³/h of the above biogas at 60% in CH₄ (v/v), the process proposed in the present invention allows a minimum production of biomethane equal to 244 Nm³/h, against the 300 Nm³/h that can be theoretically obtained, and envisages energy consumptions linked to the components listed hereunder:
- blower on the line of biogas necessary for overcoming the pressure drops in the treatment unit in the adsorption phase;
- blower on the line of the air entering the cogeneration engine;
- circulation pump of the cooling water;
- circulation pump of the heating water;
- air heater for cooling the water to 20°C;
- compression of the biomethane produced at the inlet pressure in a natural gas distribution network (7 bar);
- feeding of compressed air to the nitrogen generator and electrical consumptions of the latter;
- vacuum pump (at 100 mbar);
- auxiliary consumptions (electrovalves, control system, air conditioning, etc.).

The energy consumptions estimated for the above components are indicated in Table 2.

**Table 2 Energy consumption of the single components of the plant**

| **ENERGY CONSUMPTION [kW]** | |
|---|---|
| Biogas blower | 0.15 |
| Air blower to the cogeneration motor | 0.35 |
| Circulation pump of cooling water | 2.89 |
| Circulation pump of heating water | 1.36 |
| Air heater | 18.70 |
| Biomethane compression | 23.26 |
| Auxiliary consumptions | 10 |
| Vacuum pump (100 mbar) | 24.8 |
| Nitrogen generator | 10.3 |

The total overall consumption of the apparatus proves to be equal to 91.81 kW, against an electric power produced by the cogeneration engine equal to 226 kW. The energy consumptions of the plant are therefore completely covered by the energy production of the cogeneration engine, with a surplus of electric energy of 134 kW produced, that can be advantageously used in the production site for the functioning of other equipment or remunerated by entry into an electric energy distribution network.

## Claims

1. A process for upgrading a stream of biogas (2) produced by bacterial digestion of organic residues under anaerobic conditions, comprising at least methane and CO₂, to biomethane, said process comprising at least the following steps:
a) supplying a part (4) of said biogas stream (2) to a cogeneration system (M) and producing a heating fluid (3) and electric energy (18);
b) supplying said electric energy (18) at least to cooling means (R) and producing a cooling fluid (5);
c) supplying the remaining part (6) of said biogas stream (2) to a TPSA treatment system (S) comprising at least a first (U1) and a second treatment unit (U2), each comprising at least one solid sorbent (25); in said treatment system (S) occurring, for at least a certain operating period under regime conditions, that:
- the CO₂ present in said remaining part (6) of said biogas stream (2) is adsorbed on the solid sorbent (25) of said first treatment unit (U1) with the formation of a stream of biomethane (16');
- the solid sorbent (25) of said second treatment unit (U2) is subjected to regeneration with the formation of a stream of desorbed CO₂ (17') and a regenerated solid sorbent;
said heating fluid (3) and said cooling fluid (5) being used for regulating the temperature of said solid sorbents (25).

2. The process according to the previous claim, wherein the adsorption of said CO₂ is effected on said second treatment unit (U2) while the solid sorbent (25) of said first treatment unit (U1) is subjected to regeneration.

3. The process according to one or more of the previous claims, wherein said regeneration of the solid sorbent (25) comprises:
- desorbing said CO₂ adsorbed on said solid sorbent (25) by heating said solid sorbent (25) so as to form a stream of desorbed CO₂ (17') and a regenerated solid sorbent;
- cooling said regenerated solid sorbent.

4. The process according to one or more of the previous claims, wherein the adsorption of CO₂ is effected on said solid sorbent (25) at a temperature within the range of 20°C - 60°C.

5. The process according to one or more of claims 3-4, wherein the desorption of CO₂ is effected on said solid sorbent (25) at a temperature higher than 60°C and lower than or equal to 120°C.

6. The process according to one or more of the previous claims 3-5, wherein the desorption of CO₂ is effected at a pressure lower than 800 mbar, preferably within the range of 50-800 mbar.

7. The process according to one or more of claims 3-6, wherein said regenerated solid sorbent is cooled to a temperature within the range of 20°C - 60°C.

8. The process according to one or more of the previous claims, wherein said treatment system (S) comprises a third treatment unit (U3) comprising at least one solid sorbent (25) and wherein, for at least a certain operating period under regime conditions of said treatment system S, it occurs that:
- the CO₂ present in said remaining part (6) of said biogas stream (2) is adsorbed on the solid sorbent (25) of a first of said treatment units (U1-U3) at a temperature within the range of 20°C - 60°C with the formation of a stream of biomethane (16'-16''');
- the solid sorbent (25) of a second of said treatment units (U1-U3) is heated to a temperature higher than 60°C and lower than or equal to 120°C so as to form a stream of desorbed CO₂ (17'-17''') and a regenerated solid sorbent;
- the solid sorbent (25) of a third of said treatment units (U1-U3) is cooled to a temperature within the range of 20°C - 60°C.

9. The process according to one or more of the previous claims, wherein said desorption of CO₂ is assisted by the supply of a gaseous stripping stream (7) on said solid sorbent (25).

10. The process according to one or more of the previous claims, wherein said solid sorbent (25) is selected from: (i) carbon molecular sieves, (ii) activated carbon, (iii) one or more amine compounds, preferably alkanolamines, supported on a solid substrate, (iv) one or more carbonate salts.

11. The process according to the previous claim, wherein said solid sorbent (25) comprises at least one alkanolamine having formula OH-R₁-N-R₂R₃, wherein:
- R₁ is a group -(CH₂)ₙ wherein n is an integer from 2 to 4,
- R₂ and R₃, equal to or different from each other, are selected from: a hydrogen atom, a C₁-C₄ alkyl group or a -(CH₂)ₙOH group wherein n is an integer from 2 to 4;
said alkanolamine being supported on a solid substrate, preferably selected from alumina, silica, alumino-silicates or a combination thereof.

12. An apparatus (1) for upgrading a stream of biogas (2) produced by bacterial digestion of organic residues under anaerobic conditions, comprising at least methane and CO₂, to biomethane, comprising:
- at least one cogeneration system (M) for producing a heating fluid (3) and electric energy (18), said cogeneration system (M) being fed with at least a part (4) of said biogas stream (2);
- cooling means (R) for producing a cooling fluid (5) electrically supplied by said cogeneration system (M) ;
- at least one TPSA system (S) comprising at least a first (U1) and a second treatment unit (U2) connected in parallel to each other, each comprising at least one solid sorbent (25); said first and second treatment unit (U1, U2) being connected to said cogeneration system (M) for receiving said heating fluid (3) and to said cooling means (R) for receiving said cooling fluid (5);
- means for feeding biogas for supplying the remaining part (6) of said biogas stream (2) to at least said treatment units (U1, U2);
- means for feeding a thermal fluid for supplying said heating fluid (3) or said cooling fluid (5) to at least said treatment units (U1, U2) so as to regulate the temperature of the respective solid sorbents.

13. The apparatus (1) according to the previous claim comprising at least a third treatment unit (U3) connected in parallel to said first and second treatment units (U1, U2), said third treatment unit (U3) comprising at least one solid sorbent (25); said heating fluid (3) and said cooling fluid (5) also being fed to said further treatment unit (U3) so as to regulate the temperature of the solid sorbent (25) of said further treatment unit (U3); said remaining part (6) of said biogas stream (2) also being fed to said third treatment unit (U3).

14. The apparatus (1) according to one or more of claims 12-13, wherein said treatment unit (U1-U3) comprises:
- a main tube (22) through which said heating fluid (3) or said cooling fluid (5) flows,
- a plurality of auxiliary tubes (24), optionally parallel to each other and to said main tube (22), in contact with said main tube (22), each auxiliary tube (24) containing at least one solid sorbent (25).

15. The apparatus (1) according to one or more of claims 12-14, comprising a device (DS) for generating a gaseous stripping stream (7') connected to said treatment units (U1-U3) for assisting the desorption of CO₂ from said solid sorbents (25), said device (DS) being optionally connected to said cogeneration system (M) so as to be electrically fed thereby.

## Patentansprüche

1. Verfahren zur Aufbereitung eines Biogasstroms (2), der durch bakterielle Verdauung organischer Rückstände unter anaeroben Bedingungen erzeugt wird, umfassend wenigstens Methan und CO₂, in Biomethan, wobei das Verfahren wenigstens folgende Schritte umfasst:
a) Zuführen eines Teils (4) des Biogasstroms (2) zu einem Blockheizkraftwerksystem (M) und Erzeugen eines Heizmediums (3) und elektrischer Energie (18);
b) Zuführen der elektrischen Energie (18) wenigstens zu Kühleinrichtungen (R) und Erzeugen eines Kühlmediums (5) ;
c) Zuführen des restlichen Teils (6) des Biogasstroms (2) zu einem TPSA-Behandlungssystem (S), umfassend wenigstens eine erste (U1) und eine zweite Behandlungseinheit (U2), die jeweils wenigstens ein festes Sorptionsmittel (25) umfassen;
wobei es in dem Behandlungssystem (S) wenigstens in einer bestimmten Betriebszeit unter Regimebedingungen dazu kommt, dass:
- das in dem restlichen Teil (6) des Biogasstroms (2) vorhandene CO₂ auf dem festen Sorptionsmittel (25) der ersten Behandlungseinheit (U1) unter Bildung eines Biomethanstroms (16') absorbiert wird;
- das feste Sorptionsmittel (25) der zweiten Behandlungseinheit (U2) einer Regenerierung unter Bildung eines Stroms von desorbiertem CO₂ (17') und eines regenerierten festen Sorptionsmittels unterworfen ist;
wobei das Heizmedium (3) und das Kühlmedium (5) zur Regulierung der Temperatur der festen Sorptionsmittel (25) verwendet werden.

2. Verfahren nach dem vorstehenden Anspruch, wobei die Absorption von CO₂ in der zweiten Behandlungseinheit (U2) durchgeführt wird, während das feste Sorptionsmittel (25) der ersten Behandlungseinheit (U1) einer Regenerierung unterworfen ist.

3. Verfahren nach einem oder mehreren der vorstehenden Ansprüche, wobei die Regenerierung des festen Sorptionsmittels (25) Folgendes umfasst:
- Desorbieren des auf dem festen Sorptionsmittel (25) absorbierten CO₂ durch Erhitzen des festen Sorptionsmittels (25), damit ein Strom von desorbiertem CO₂ (17') und ein regeneriertes festes Sorptionsmittel gebildet wird;
- Kühlen des regenerierten festen Sorptionsmittels.

4. Verfahren nach einem oder mehreren der vorstehenden Ansprüche, wobei die Absorption von CO₂ auf dem festen Sorptionsmittel (25) bei einer Temperatur im Bereich von 20 °C - 60 °C erfolgt.

5. Verfahren nach einem oder mehreren der Ansprüche 3-4, wobei die Desorption von CO₂ auf dem festen Sorptionsmittel (25) bei einer Temperatur von über 60 °C und unter oder gleich 120 °C erfolgt.

6. Verfahren nach einem oder mehreren der vorstehenden Ansprüche 3-5, wobei die Desorption von CO₂ bei einem Druck von unter 800 mbar, vorzugsweise im Bereich von 50-800 mbar, erfolgt.

7. Verfahren nach einem oder mehreren der Ansprüche 3-6, wobei das regenerierte feste Sorptionsmittel auf eine Temperatur im Bereich von 20 °C - 60 °C abgekühlt wird.

8. Verfahren nach einem oder mehreren der vorstehenden Ansprüche, wobei das Behandlungssystem (S) eine dritte Behandlungseinheit (U3) umfasst, die wenigstens ein festes Sorptionsmittel (25) umfasst, und wobei es wenigstens in einer bestimmten Betriebszeit unter Regimebedingungen des Behandlungssystems S dazu kommt, dass:
- das in dem restlichen Teil (6) des Biogasstroms (2) vorhandene CO₂ auf dem festen Sorptionsmittel (25) einer ersten der Behandlungseinheiten (U1-U3) bei einer Temperatur im Bereich von 20 °C - 60 °C unter Bildung eines Biomethanstroms (16'-16''') absorbiert wird;
- das feste Sorptionsmittel (25) einer zweiten der Behandlungseinheiten (U1-U3) auf eine Temperatur von über 60 °C und unter oder gleich 120 °C erhitzt wird, um einen Strom von desorbiertem CO₂ (17'-17''') und ein regeneriertes festes Sorptionsmittel zu bilden;
- das feste Sorptionsmittel (25) einer dritten der Behandlungseinheiten (U1-U3) auf eine Temperatur im Bereich von 20 °C - 60 °C abgekühlt wird.

9. Verfahren nach einem oder mehreren der vorstehenden Ansprüche, wobei die Desorption von CO₂ durch die Zufuhr eines gasförmigen Strippstroms (7) auf das feste Sorptionsmittel (25) unterstützt wird.

10. Verfahren nach einem oder mehreren der vorstehenden Ansprüche, wobei das feste Sorptionsmittel (25) ausgewählt ist aus: (i) Kohlenstoffmolekülsiebe, (ii) Aktivkohle, (iii) eine oder mehrere Aminverbindungen, vorzugsweise Alkanolamine, gehalten auf einem festen Substrat, (iv) ein oder mehrere Carbonatsalze.

11. Verfahren nach dem vorstehenden Anspruch, wobei das feste Sorptionsmittel (25) wenigstens ein Alkanolamin mit der Formel OH-R₁-N-R₂R₃ umfasst, wobei:
- R₁ eine -(CH₂)ₙ-Gruppe ist, wobei n eine ganze Zahl von 2 bis 4 ist,
- R₂ und R₃, gleich oder verschieden voneinander, ausgewählt sind aus: einem Wasserstoffatom, einer C₁-C₄ Alkylgruppe oder einer -(CH₂)ₙOH-Gruppe, wobei n eine ganze Zahl von 2 bis 4 ist;
wobei Alkanolamin auf einem festen Substrat gehalten ist, vorzugsweise ausgewählt aus Aluminiumoxid, Siliciumdioxid, Alumosilikaten oder einer Kombination davon.

12. Vorrichtung (1) zur Aufbereitung eines Biogasstroms (2), der durch bakterielle Verdauung organischer Rückstände unter anaeroben Bedingungen erzeugt wird, umfassend wenigstens Methan und CO₂, in Biomethan, umfassend:
- wenigstens ein Blockheizkraftwerksystem (M) zum Erzeugen eines Heizmediums (3) und elektrischer Energie (18), wobei das Blockheizkraftwerksystem (M) mit wenigstens einem Teil (4) des Biogasstroms (2) gespeist wird;
- Kühleinrichtungen (R) zum Erzeugen eines von dem Blockheizkraftwerksystem (M) elektrisch zugeführten Kühlmediums (5);
- wenigstens ein TPSA-System (S), umfassend wenigstens eine erste (U1) und eine zweite Behandlungseinheit (U2), die zueinander parallel geschaltet sind und jeweils wenigstens ein festes Sorptionsmittel (25) umfassen;
wobei die erste und die zweite Behandlungseinheit (U1, U2) mit dem Blockheizkraftwerksystem (M) verbunden sind, um das Heizmedium (3) aufzunehmen und mit den Kühleinrichtungen (R), um das Kühlmedium (5) aufzunehmen;
- Mittel zum Einspeisen von Biogas, um den restlichen Teil (6) des Biogasstroms (2) wenigstens den Behandlungseinheiten (U1, U2) zuzuführen;
- Mittel zum Einspeisen eines thermalen Mediums, um das Heizmedium (3) oder das Kühlmedium (5) wenigstens den Behandlungseinheiten (U1, U2) zuzuführen, um die Temperatur des jeweiligen festen Sorptionsmittels zu regulieren.

13. Vorrichtung (1) nach dem vorstehenden Anspruch, umfassend wenigstens eine dritte Behandlungseinheit (U3), die zu der ersten und zu der zweiten Behandlungseinheiten (U1, U2) parallel geschaltet ist, wobei die dritte Behandlungseinheit (U3) wenigstens ein festes Sorptionsmittel (25) umfasst; wobei das Heizmedium (3) und das Kühlmedium (5) außerdem zu der weiteren Behandlungseinheit (U3) zugeführt werden, um die Temperatur des festen Sorptionsmittels (25) der weiteren Behandlungseinheit (U3) zu regeln; wobei der restliche Teil (6) des Biogasstroms (2) außerdem in die dritte Behandlungseinheit (U3) eingespeist wird.

14. Vorrichtung (1) nach einem oder mehreren der Ansprüche 12-13, wobei die Behandlungseinheit (U1-U3) Folgendes umfasst:
- ein Hauptrohr (22), durch welches das Heizmedium (3) oder das Kühlmedium (5) fließt,
- eine Mehrzahl von Hilfsrohren (24), die wahlweise zueinander und zum Hauptrohr (22) parallel und in Kontakt mit dem Hauptrohr (22) sind, wobei jedes Hilfsrohr (24) wenigstens ein festes Sorptionsmittel (25) enthält.

15. Vorrichtung (1) nach einem oder mehreren der Ansprüche 12-14, umfassend eine Einrichtung (DS) zum Erzeugen eines gasförmigen Strippstroms (7'), die mit den Behandlungseinheiten (U1-U3) verbunden ist, um die Desorption von CO₂ von den festen Sorptionsmitteln (25) zu unterstützen, wobei die Einrichtung (DS) wahlweise mit dem Blockheizkraftwerksystem (M) verbunden ist, um hiervon elektrisch gespeist zu werden.

## Revendications

1. Procédé pour la valorisation d'un flux de biogaz (2) produit par digestion bactérienne de résidus organiques dans des conditions anaérobies, comprenant au moins du méthane et du CO₂, en biométhane, ledit procédé comprenant au moins les étapes suivantes :
a) l'alimentation d'une partie (4) dudit flux de biogaz (2) vers un système de cogénération (M) et la production d'un fluide de chauffage (3) et d'énergie électrique (18) ;
b) l'alimentation de ladite énergie électrique (18) au moins vers des moyens de refroidissement (R) et la production d'un fluide de refroidissement (5) ;
c) l'alimentation de la partie restante (6) dudit flux de biogaz (2) vers un système de traitement TPSA (S) comprenant au moins une première (U1) et une deuxième unité de traitement (U2), chacune comprenant au moins un sorbant solide (25) ;
dans ledit système de traitement (S) se produisant, pendant au moins une certaine période de fonctionnement dans des conditions de régime, ce qui suit :
- le CO₂ présent dans ladite partie restante (6) dudit flux de biogaz (2) est adsorbé sur le sorbant solide (25) de ladite première unité de traitement (U1) avec la formation d'un flux de biométhane (16') ;
- le sorbant solide (25) de ladite deuxième unité de traitement (U2) est soumis à une régénération avec la formation d'un flux de CO₂ désorbé (17') et d'un sorbant solide régénéré ;
ledit fluide de chauffage (3) et ledit fluide de refroidissement (5) étant utilisés pour réguler la température desdits sorbants solides (25).

2. Procédé selon la revendication précédente, dans lequel l'adsorption dudit CO₂ est effectuée sur ladite deuxième unité de traitement (U2) alors que le sorbant solide (25) de ladite première unité de traitement (U1) est soumis à une régénération.

3. Procédé selon une ou plusieurs des revendications précédentes, dans lequel ladite régénération du sorbant solide (25) comprend :
- la désorption dudit CO₂ adsorbé sur ledit sorbant solide (25) en chauffant ledit sorbant solide (25) de manière à former un flux de CO₂ désorbé (17') et un sorbant solide régénéré ;
- le refroidissement dudit sorbant solide régénéré.

4. Procédé selon une ou plusieurs des revendications précédentes, dans lequel l'adsorption de CO₂ est effectuée sur ledit sorbant solide (25) à une température dans la plage de 20°C à 60°C.

5. Procédé selon une ou plusieurs des revendications 3-4, dans lequel la désorption de CO₂ est effectuée sur ledit sorbant solide (25) à une température supérieure à 60°C et inférieure ou égale à 120°C.

6. Procédé selon une ou plusieurs des revendications précédentes 3 à 5, dans lequel la désorption de CO₂ est effectuée à une pression inférieure à 800 mbar, de préférence dans la plage de 50 à 800 mbar.

7. Procédé selon une ou plusieurs des revendications 3 à 6, dans lequel ledit sorbant solide régénéré est refroidi à une température dans la plage de 20°C à 60°C.

8. Procédé selon une ou plusieurs des revendications précédentes, dans lequel ledit système de traitement (S) comprend une troisième unité de traitement (U3) comprenant au moins un sorbant solide (25) et dans lequel, pendant au moins une certaine période de fonctionnement dans des conditions de régime dudit système de traitement S, il se produit ce qui suit :
- le CO₂ présent dans ladite partie restante (6) dudit flux de biogaz (2) est adsorbé sur le sorbant solide (25) d'une première desdites unités de traitement (U1-U3) à une température dans la plage de 20°C à 60°C avec la formation d'un flux de biométhane (16'-16''') ; ;
- le sorbant solide (25) d'une deuxième desdites unités de traitement (U1-U3) est chauffé à une température supérieure à 60°C et inférieure ou égale à 120°C de manière à former un flux de CO₂ désorbé (17'-17''') et un sorbant solide régénéré ;
- le sorbant solide (25) d'une troisième desdites unités de traitement (U1-U3) est refroidi à une température dans la plage de 20°C - 60°C.

9. Procédé selon une ou plusieurs des revendications précédentes, dans lequel ladite désorption de CO₂ est assistée par l'alimentation d'un flux d'épuisement gazeux (7) sur ledit sorbant solide (25).

10. Procédé selon une ou plusieurs des revendications précédentes, dans lequel ledit sorbant solide (25) est sélectionné parmi : (i) des tamis moléculaires de carbone, (ii) du charbon actif, (iii) un ou plusieurs composés aminés, de préférence des alcanolamines, supportés sur un substrat solide, (iv) un ou plusieurs sels de carbonates.

11. Procédé selon la revendication précédente, dans lequel ledit sorbant solide (25) comprend au moins une alcanolamine ayant la formule OH-R₁-N-R₂R₃, dans laquelle:
- R₁ est un groupe -(CH₂)ₙ dans lequel n est un entier de 2 à 4,
- R₂ et R₃, identiques ou différents entre eux, sont sélectionnés parmi : un atome d'hydrogène, un groupe alkyle en C₁-C₄ ou un groupe -(CH₂)ₙOH dans lequel n est un entier de 2 à 4 ;
ladite alcanolamine étant supportée sur un substrat solide, de préférence sélectionné parmi l'alumine, la silice, des aluminosilicates ou une combinaison de ceux-ci.

12. Appareil (1) pour la valorisation d'un flux de biogaz (2) produit par digestion bactérienne de résidus organiques dans des conditions anaérobies, comprenant au moins du méthane et du CO₂, en biométhane, comprenant :
- au moins un système de cogénération (M) pour produire un fluide de chauffage (3) et de l'énergie électrique (18), ledit système de cogénération (M) étant alimenté avec au moins une partie (4) dudit flux de biogaz (2) ;
- des moyens de refroidissement (R) pour produire un fluide de refroidissement (5) alimenté électriquement par ledit système de cogénération (M) ;
- au moins un système TPSA (S) comprenant au moins une première (U1) et une deuxième unité de traitement (U2) connectées en parallèle entre elles, comprenant chacune au moins un sorbant solide (25) ; lesdites première et deuxième unités de traitement (U1, U2) étant connectées audit système de cogénération (M) pour recevoir ledit fluide de chauffage (3) et auxdits moyens de refroidissement (R) pour recevoir ledit fluide de refroidissement (5) ;
- des moyens d'alimentation de biogaz pour l'alimentation de la partie restante (6) dudit flux de biogaz (2) vers au moins lesdites unités de traitement (U1, U2) ;
- des moyens d'alimentation d'un fluide thermique pour alimenter ledit fluide de chauffage (3) ou ledit fluide de refroidissement (5) vers au moins lesdites unités de traitement (U1, U2) de manière à réguler la température des sorbants solides respectifs.

13. Appareil (1) selon la revendication précédente comprenant au moins une troisième unité de traitement (U3) connectée en parallèle avec lesdites première et deuxième unités de traitement (U1, U2), ladite troisième unité de traitement (U3) comprenant au moins un sorbant solide (25) ; ledit fluide de chauffage (3) et ledit fluide de refroidissement (5) étant également alimentés vers ladite autre unité de traitement (U3) de manière à réguler la température du sorbant solide (25) de ladite autre unité de traitement (U3) ; ladite partie restante (6) dudit flux de biogaz (2) étant également alimentée vers ladite troisième unité de traitement (U3).

14. Appareil (1) selon une ou plusieurs des revendications 12 à 13, dans lequel ladite unité de traitement (U1-U3) comprend :
- un tube principal (22) à travers lequel ledit fluide de chauffage (3) ou ledit fluide de refroidissement (5) s'écoule,
- une pluralité de tubes auxiliaires (24), facultativement parallèles entre eux et audit tube principal (22), en contact avec ledit tube principal (22), chaque tube auxiliaire (24) contenant au moins un sorbant solide (25).

15. Appareil (1) selon une ou plusieurs des revendications 12 à 14, comprenant un dispositif (DS) pour générer un flux d'épuisement gazeux (7'), connecté auxdites unités de traitement (U1-U3) pour assister la désorption de CO₂ à partir desdits sorbants solides (25), ledit dispositif (DS) étant facultativement connecté audit système de cogénération (M) de manière à être alimenté électriquement par celui-ci.
